# EUROPEAN PATENT APPLICATION

(11) **EP 4 822 019 A1**
(43) Date of publication of application: **14.10.2026**
(21) Application number: 24899795.9
(22) Date of filing: 03.12.2024
(51) Int. Cl.: C12N 9/26, C12N 15/56, C12N 15/63, C12P 13/04

(54) **USE OF AGLA GENE IN PREPARATION OF L-AMINO ACID**

(30) Priority: 08.12.2023 CN 202311679969
(71) Applicant: Ningxia Eppen Biotech Co. Ltd, Ningxia 750100 (CN)
(72) Inventor: WEI, Aiying, Yinchuan, Ningxia 750100 (CN); MENG, Gang, Yinchuan, Ningxia 750100 (CN); ZHOU, Xiaoqun, Yinchuan, Ningxia 750100 (CN); ZHAO, Chunguang, Yinchuan, Ningxia 750100 (CN); MA, Fengyong, Yinchuan, Ningxia 750100 (CN); JIA, Huiping, Yinchuan, Ningxia 750100 (CN); SU, Houbo, Yinchuan, Ningxia 750100 (CN)
(74) Representative: Winter, Brandl - Partnerschaft mbB
(86) International application number: PCT/CN2024/136335
(87) International publication number: WO 2025/119157

(57) **Abstract**

Disclosed are an α-glucosidase gene and the use of α-glucosidase or a substance that regulates the activity and/or content of α-glucosidase in the preparation of L-amino acids.

## Description

### TECHNICAL FIELD

The present application relates to the use of an aglA gene in the preparation of L-amino acids in the field of biotechnology.

### BACKGROUND

L-amino acids have been used in animal feed, pharmaceutical, and cosmetic industries, and are mainly produced by fermentation using genus Corynebacterium or genus Escherichia. At present, the main problems in the production of L-amino acids are low acid production and low conversion. How to fully improve substrate utilization efficiency so as to increase the yield of L-amino acids during fermentation remains one of the key issues to be solved.

### SUMMARY

The technical problem to be solved by the present application is how to increase the yield of L-amino acids. The technical problem to be solved is not limited to the described technical subject matter, and those skilled in the art can clearly understand other technical subject matters not mentioned herein from the following description.

To solve the above technical problem, the present application provides the following technical solutions:
The present application provides the following uses:
U1) use of α-glucosidase in the preparation of L-amino acids;
U2) use of a substance that regulates the activity of the α-glucosidase in the preparation of L-amino acids; and
U3) use of a substance that regulates the content of the α-glucosidase in the preparation of L-amino acids.

The above α-glucosidase comprises A1), A2), or A3):
A1) a protein having an amino acid sequence of SEQ ID NO.5;
A2) a protein derived from A1) or having more than 45% identity with the protein shown in A1) and having the same function, obtained by substitution and/or deletion and/or addition of amino acid residues to the amino acid sequence shown in SEQ ID NO.5; and
A3) a fusion protein having the same function obtained by linking a tag to the N-terminus and/or C-terminus of A1) or A2).

The above-mentioned more than 45% identity comprises 47%-65% identity, more than 65% identity, more than 70% identity, or more than 80% identity. The 47%-65% identity specifically comprises 47% identity, 54% identity, 57% identity, or 62% identity.

In the above uses, the more than 45% identity may be at least 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% identity. In the above uses, the 47%-65% identity may be 47%, 48%, 49%, 50%, 51%, 52%, 53%, 54%, 55%, 56%, 57%, 58%, 59%, 60%, 61%, 62%, 63%, 64%, or 65% identity. In the above uses, the more than 65% identity may be at least 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% identity. In the above uses, the more than 70% identity may be at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% identity. In the above uses, the more than 80% identity may be at least 80%, 81%, 82%, 83%, 84%, 85%, 86%, 87%, 88%, 89%, 90%, 91%, 92%, 93%, 94%, 95%, 96%, 97%, 98%, 99%, or 100% identity.

Herein, identity refers to the identity of an amino acid sequence or a nucleotide sequence. The identity of an amino acid sequence can be determined using a homology search site on the internet, such as the BLAST webpage on the NCBI homepage. For example, in advanced BLAST 2.1, blastp can be used as the program, the Expect value can be set to 10, all Filters can be set to OFF, BLOSUM62 can be used as the Matrix, the Gap existence cost, Per residue gap cost, and Lambda ratio can be set to 11, 1, and 0.85 (default values), respectively, and a search can be performed to calculate the identity of the amino acid sequence, thereby obtaining the identity value (%).

In the present application, the regulation may comprise upregulating or enhancing or increasing the expression level of the aforementioned protein, or may comprise downregulating or inhibiting or decreasing the expression level of the aforementioned protein.

In the present application, the substance comprises a substance that performs at least one of the following six types of regulation: 1) regulation at the transcriptional level of the gene; 2) regulation after transcription of the gene (i.e., regulation of splicing or processing of the primary transcript of the gene); 3) regulation of RNA transport of the gene (i.e., regulation of transport of the mRNA of the gene from the nucleus to the cytoplasm); 4) regulation of translation of the gene; 5) regulation of mRNA degradation of the gene; 6) regulation after translation of the gene (i.e., regulation of the activity of the protein translated from the gene).

The L-amino acids in the above uses comprise polar neutral amino acids, acidic amino acids, basic amino acids, and/or nonpolar hydrophobic amino acids. The basic amino acids comprise lysine, arginine, and/or histidine. The nonpolar hydrophobic amino acids comprise glycine, alanine, valine, leucine, isoleucine, phenylalanine, and/or proline. The polar neutral amino acids comprise tryptophan, tyrosine, serine, threonine, cysteine, methionine, glutamine, or asparagine. The acidic amino acids comprise glutamic acid or aspartic acid.

In the above uses, the protein in A2) comprises a protein having an amino acid sequence of SEQ ID NO.1, SEQ ID NO.2, SEQ ID NO.3, or SEQ ID NO.4.

In the above uses, the substance is a biological material related to the above protein, and the biological material comprises B1), B2), B3), B4), or B5):
B1) a nucleic acid molecule encoding the protein; B2) an expression cassette containing the nucleic acid molecule of B1);
B3) a recombinant vector containing the nucleic acid molecule of B1), or a recombinant vector containing the expression cassette of B2); B4) a recombinant microorganism containing the nucleic acid molecule of B1), or a recombinant microorganism containing the expression cassette of B2), or a recombinant microorganism containing the recombinant vector of B3); and B5) a whole-cell catalyst containing the nucleic acid molecule of B1), or a whole-cell catalyst containing the expression cassette of B2), or a whole-cell catalyst containing the recombinant vector of B3), or a whole-cell catalyst containing the recombinant microorganism of B4).

The expression cassette in the above biological material refers to DNA capable of expressing the above protein in a host cell. The expression cassette may further comprise a single-stranded or double-stranded nucleic acid molecule containing all regulatory sequences necessary for the nucleic acid molecule expressing any one of the above proteins. The regulatory sequences can direct the expression of any one of the above proteins from the coding sequence in a suitable host cell under their compatible conditions. The regulatory sequences comprise, but are not limited to, a leader sequence, a polyadenylation sequence, a propeptide sequence, a promoter, a signal sequence, and a transcription terminator. At a minimum, the regulatory sequences should comprise a promoter and transcription and translation termination signals. In order to introduce specific restriction enzyme sites into the vector for linking the regulatory sequences with the coding region of the nucleic acid sequence encoding the protein, regulatory sequences with adapters may be provided. The regulatory sequences may be a suitable promoter sequence, i.e., a nucleic acid sequence recognizable by the host cell expressing the nucleic acid sequence. The promoter sequence contains transcription regulatory sequences that mediate protein expression. The promoter may be any nucleic acid sequence having transcriptional activity in the selected host cell, including mutated, truncated, and hybrid promoters, and may be derived from a gene encoding an extracellular or intracellular protein homologous or heterologous to the host cell. The regulatory sequences may also be a suitable transcription termination sequence, i.e., a sequence recognizable by the host cell to terminate transcription. The termination sequence may be operably linked to the 3' end of the nucleic acid sequence encoding the protein. Any terminator functional in the selected host cell may be used in the present application. The regulatory sequences may also be a suitable leader sequence, i.e., an mRNA untranslated region important for translation in the host cell. The leader sequence may be operably linked to the 5' end of the nucleic acid sequence encoding the protein. Any leader sequence functional in the selected host cell may be used in the present application. The regulatory sequences may also be a signal peptide coding region encoding an amino acid sequence linked to the amino terminus of the protein that can direct the encoded protein into the cellular secretion pathway. Any signal peptide coding region capable of directing the expressed protein into the secretion pathway of the host cell used may be used in the present application. It may also be necessary to add regulatory sequences that can regulate protein expression according to the growth conditions of the host cell. Examples of regulatory systems are those that can respond to chemical or physical stimuli (including in the presence of regulatory compounds) to turn on or off gene expression. Other examples of regulatory sequences are those that enable gene amplification. In these examples, the nucleic acid sequence encoding the protein should be operably linked to the regulatory sequences.

The recombinant vector may comprise a nucleic acid molecule encoding the above protein, a promoter, and transcription and translation termination signals. When preparing the recombinant vector, the nucleic acid molecule encoding the above protein may be positioned in the vector so as to be operably linked to appropriate expression regulatory sequences. The recombinant vector may be any vector (e.g., a plasmid or a virus) that facilitates recombinant DNA manipulation and expression of the nucleic acid sequence. The choice of vector usually depends on the compatibility of the vector with the host cell into which it is to be introduced. The vector may be a linear or circular plasmid. The vector may be an autonomously replicating vector (i.e., a complete structure existing extrachromosomally that can replicate independently of the chromosome), such as a plasmid, an extrachromosomal element, a minichromosome, or an artificial chromosome. The vector may contain any mechanism that ensures self-replication. Alternatively, the vector is one that, when introduced into a host cell, integrates into the genome and replicates along with the chromosome into which it is integrated. Furthermore, a single vector or plasmid, or two or more vectors or plasmids, or transposons, that collectively contain all DNA to be introduced into the host cell genome may be used. The vector contains one or more selection markers for selecting transformed cells. A selection marker is a gene whose product confers resistance to a biocide or virus, resistance to a heavy metal, or confers prototrophy on an auxotroph, etc. Examples of bacterial selection markers comprise the dal gene of bacillus subtilis or bacillus licheniformis, or resistance markers for antibiotics such as ampicillin, kanamycin, chloramphenicol, or tetracycline. The vector contains elements that enable stable integration of the vector into the host cell genome or ensure autonomous replication of the vector in the cell independent of the cell genome. In the case of autonomous replication, the vector may also contain an origin of replication that enables the vector to replicate autonomously in the target host cell. The origin of replication may carry a mutation that makes it temperature-sensitive in the host cell (see, for example, fEhrlich, 1978, proceedings of the national academy of sciences 75: 1433). More than one copy of the nucleic acid molecule of the present application encoding any one of the above proteins may be inserted into the host cell to increase the yield of the gene product. The increase in the copy number of the nucleic acid molecule may be achieved by inserting at least one additional copy of the nucleic acid molecule into the host cell genome, or by inserting an amplifiable selection marker together with the nucleic acid molecule, and by culturing the cells in the presence of a suitable selection agent, selecting cells containing an amplified copy of the selection marker gene and thus containing an additional copy of the nucleic acid molecule. The operations for linking the above elements to construct the recombinant expression vector of the present application are well known to those skilled in the art (see, for example, Sambrook et al., molecular cloning: a laboratory manual, second edition, cold spring harbor laboratory press, cold spring harbor, new york, 1989).

The term "operably linked" is defined herein as a configuration in which a regulatory sequence is positioned at an appropriate position relative to the coding sequence of a DNA sequence so that the regulatory sequence directs the expression of the protein.

The whole-cell catalyst in B5) above comprises cells, and the cells comprise microbial cells, plant cells, or animal cells. The animal cells do not comprise human germ cells, fertilized eggs, embryos, or individuals. The animal cells comprise somatic cells or cell lines of animals.

The nucleic acid molecule in B1) above comprises any one of C1) to C6):
C1) a cDNA molecule or DNA molecule whose coding sequence comprises SEQ ID NO.6;
C2) a coding gene whose coding sequence comprises an amino acid sequence of SEQ ID NO.1;
C3) a coding gene whose coding sequence comprises an amino acid sequence of SEQ ID NO.2;
C4) a coding gene whose coding sequence comprises an amino acid sequence of SEQ ID NO.3;
C5) a coding gene whose coding sequence comprises an amino acid sequence of SEQ ID NO.4; and
C6) a cDNA molecule or DNA molecule that hybridizes with the cDNA or DNA molecule of the coding gene defined by C1) or C2) or C3) or C4) or C5) and encodes a protein having the same function.

The above protein also falls within the scope of protection of the present application.

The above biological material also falls within the scope of protection of the present application.

The present application also provides a method for producing L-amino acids, comprising fermenting and preparing L-amino acids using the aforementioned protein or the aforementioned biological material.

The L-amino acids in the above method comprise L-glutamic acid, L-lysine, or L-threonine.

The use of the above protein or the above biological material in the preparation of food, feed, medicine, fertilizer, or daily chemical products containing L-amino acids also falls within the scope of protection of the present application.

### Beneficial effects

The present application has been experimentally proven that, compared with the target microorganism wild-type Corynebacterium glutamicum ATCC13032 and the L-lysine-producing strain YP097158, the L-lysine yield of the recombinant Corynebacterium glutamicum YPL-aglA-1, YPL-aglA-2, YPL-aglA-3, and YPL-aglA-4 overexpressing the aglA-5 gene is significantly increased. Compared with the target microorganism wild-type Escherichia coli W3110 and Escherichia coli CGMCC No.25404, the L-threonine yield of the recombinant Corynebacterium glutamicum YPT-aglA-1, YPT-aglA-2, YPT-aglA-3, and YPT-aglA-4 overexpressing the aglA-5 gene is significantly increased. Compared with the target microorganism wild-type Corynebacterium glutamicum ATCC13869 and the L-lysine-producing strain CGMCC No.21220, the L-glutamic acid yield of the recombinant Corynebacterium glutamicum YPG-aglA-1, YPG-aglA-2, YPG-aglA-3, and YPG-aglA-4 overexpressing the aglA-5 gene is significantly increased.

### Deposit description

Strain name: Corynebacterium glutamicum
Latin name: Corynebacterium glutamicum
Strain number: YP097158
Depositary: China General Microbiological Culture Collection Center
Abbreviation of depositary: CGMCC
Address: No.3, Yard 1, Beichen West Road, Chaoyang District, Beijing
Date of deposit: August 16, 2016
Accession number: CGMCC No.12856.
Strain name: Corynebacterium glutamicum
Latin name: Corynebacterium glutamicum
Strain number: YPGLU001
Depositary: China General Microbiological Culture Collection Center
Abbreviation of depositary: CGMCC
Address: No.3, Yard 1, Beichen West Road, Chaoyang District, Beijing
Date of deposit: November 23, 2020
Accession number: CGMCC No.21220.

### DETAILED DESCRIPTION

The present application is further described in detail below in conjunction with specific embodiments. The examples provided are only for the purpose of illustrating the present application, and not for limiting the scope of the present application. The examples provided below can serve as a guide for those of ordinary skill in the art to make further improvements, and do not constitute any limitation on the present application in any way.

The experimental methods in the following examples are conventional methods unless otherwise specified, and are performed according to the techniques or conditions described in the literature in the art or according to the product instructions. The materials, reagents, etc. used in the following examples are commercially available unless otherwise specified.

Unless the content explicitly indicates otherwise, the singular forms "a", "an", and "one" as used in this specification and the appended claims comprise plural referents. As used herein, the term "nucleotide" refers to a monomeric unit of a polynucleotide, which consists of a heterocyclic base, a sugar, and one or more phosphate groups. Naturally occurring bases (guanine (G), adenine (A), cytosine (C), thymine (T), and uracil (U)) are usually derivatives of purines or pyrimidines, but it should be understood that both naturally and non-naturally occurring base analogs are also included. Naturally occurring sugars are pentoses (five-carbon sugars), deoxyribose (which forms DNA), or ribose (which forms RNA), but it should be understood that both naturally and non-naturally occurring sugar analogs are also included. Nucleic acids are usually linked by phosphodiester bonds to form nucleic acids or polynucleotides, and may also be linked by other bonds known in the art, such as phosphorothioate bonds.

As used herein, the terms "polypeptide" and "protein" are used interchangeably and refer to a polymer of amino acid residues. As used herein, the term "vector" refers to a nucleic acid molecule capable of transporting another nucleic acid (i.e., a polynucleotide sequence) linked thereto. As used herein, the term "fermentation" broadly refers to the conversion of organic materials into target substances by host cells, for example, by culturing recombinant host cells in a medium comprising a carbon source so that the recombinant host cells convert the carbon source into lysine.

Wild-type Corynebacterium glutamicum ATCC13032 strain: a product of the American type culture collection (ATCC).

The Corynebacterium glutamicum YP097158 used in the following examples is described in the Chinese patent document with authorization number "CN110607313B" and entitled "Recombinant bacterial strain for high-yield production of L-lysine, and construction method and use of recombinant bacterial strain", with strain number YP097158, which is deposited with the China General Microbiological Culture Collection Center on August 16, 2016, under deposit number CGMCC No.12856. Hereinafter referred to as Corynebacterium glutamicum CGMCC No.YP097158.

The Corynebacterium glutamicum CGMCC No.21220 used in the following examples is described in the Chinese patent document with authorization number "CN112725253B" and entitled "Recombinant strain for modifying gene BBD29-14900 as well as construction method and use of recombinant strain", with strain number YPGLU001, which is deposited with the China General Microbiological Culture Collection Center on November 23, 2020, under deposit number CGMCC No.21220. Hereinafter referred to as Corynebacterium glutamicum CGMCC No.21220.

The following examples use GraphPad Prism 8 statistical software for data processing. The experimental results are expressed as mean + standard deviation, and One-way ANOVA test is used. P < 0.01 indicates an extremely significant difference.

### Example 1: Construction of engineered strains containing aglA vectors

### I. Construction of aglA gene vectors

In order to study the effect of aglA on isomaltose utilization, first, using the aglA gene sequence in the genome of Bifidobacterium adolescentis published by NCBI as a template, the protein function and structure are analyzed, and 5 aglA genes are designed and synthesized: aglA-1 (nucleotide sequence of SEQ ID NO.7, encoding the amino acid sequence shown in SEQ ID NO.1), aglA-2 (nucleotide sequence of SEQ ID NO.8, encoding the amino acid sequence shown in SEQ ID NO.2), aglA-3 (nucleotide sequence of SEQ ID NO.9, encoding the amino acid sequence shown in SEQ ID NO.3), aglA-4 (nucleotide sequence of SEQ ID NO.10, encoding the amino acid sequence shown in SEQ ID NO.4), and aglA-5 (nucleotide sequence as shown in SEQ ID NO.6, encoding the amino acid sequence shown in SEQ ID NO.5).

The above 5 synthesized aglA genes (synthesized with the H36 promoter, SEQ ID NO.50) and the expression vector pXMJ19 (purchased from Biovector, catalog number Biovector pXMJ19, containing chloramphenicol resistance) digested with XbalI and BamHI and recovered are ligated using NEBuilder enzyme (purchased from NEB) at 50°C for 30 min. The ligation products are transformed into DH5α competent cells, plated on 2-YT agar plates containing chloramphenicol (34 mg/L), and cultured at 37°C for 12 h. The grown monoclones are identified by PCR using primers MJ19-F (5'-GCGGATAACAATTTCACACAG-3', SEQ ID NO.11) / MJ19-R (5'-CTCTCATCCGCCAAAACAG-3', SEQ ID NO.12). PCR amplification system: 2× Premix rTaq 12.5 µL, each primer (10 pM) 1 µL, and ddH₂O added to a total volume of 25 µL. PCR amplification program: pre-denaturation at 94°C for 15 min, denaturation at 94°C for 30 s, annealing at 58°C for 30 s, extension at 72°C for 2 min (30 cycles), and over-extension at 72°C for 10 min.

The identified positive strains are separately extracted for plasmids and sent for sequencing. The correctly sequenced plasmids are named pXMJ19-aglA-1 (containing the gene sequence shown in SEQ ID NO.7), pXMJ19-aglA-2 (containing the gene sequence shown in SEQ ID NO.8), pXMJ19-aglA-3 (containing the gene sequence shown in SEQ ID NO.9), pXMJ19-aglA-4 (containing the gene sequence shown in SEQ ID NO.10), and pXMJ19-aglA-5 (containing the gene sequence shown in SEQ ID NO.6), respectively.

pXMJ19-aglA-1 is a recombinant expression vector obtained by replacing the sequence between the XbalI and BamHI recognition sites of pXMJ19 with SEQ ID NO.7 while keeping other nucleotide sequences unchanged, and the vector expresses a protein having the amino acid sequence of SEQ ID NO.1. pXMJ19-aglA-2 is a recombinant expression vector obtained by replacing the sequence between the XbalI and BamHI recognition sites of pXMJ19 with SEQ ID NO.8 while keeping other nucleotide sequences unchanged, and the vector expresses a protein having the amino acid sequence of SEQ ID NO.2. pXMJ19-aglA-3 is a recombinant expression vector obtained by replacing the sequence between the XbalI and BamHI recognition sites of pXMJ19 with SEQ ID NO.9 while keeping other nucleotide sequences unchanged, and the vector expresses a protein having the amino acid sequence of SEQ ID NO.3. pXMJ19-aglA-4 is a recombinant expression vector obtained by replacing the sequence between the XbalI and BamHI recognition sites of pXMJ19 with SEQ ID NO.10 while keeping other nucleotide sequences unchanged, and the vector expresses a protein having the amino acid sequence of SEQ ID NO.4.

pXMJ19-aglA-5 is a recombinant expression vector obtained by replacing the sequence between the XbalI and BamHI recognition sites of pXMJ19 with SEQ ID NO.6 while keeping other nucleotide sequences unchanged, and the vector expresses a protein having the amino acid sequence of SEQ ID NO.5.

### II. Construction of ATCC13032 strains containing aglA gene vectors

Culture plates containing chloramphenicol (34 mg/L): the solvent is water, and the solutes and their concentrations are sucrose 10 g/L, polypeptone 10 g/L, beef extract 10 g/L, yeast powder 5 g/L, urea 2 g/L, sodium chloride 2.5 g/L, agar powder 18 g/L, chloramphenicol 34 mg/L, pH 7.0.

Rich medium: the solvent is water, and the solutes and their concentrations are glucose 25 g/L, isomaltose 5 g/L, (NH4)₂SO₄ 2 g/L, H₃PO₄ 0.5 g/L, KCl 0.8 g/L, MgSO₄•7H₂O 0.8 g/L, FeSO₄•7H₂O 0.05 g/L, MnSO₄•H₂O 0.05 g/L, FM902 yeast powder 1.5 g/L, corn steep liquor 5 g/L, molasses 17 g/L, betaine 0.5 g/L, citric acid 2 g/L, VH 20 mg/L, VB₁ 1.5 mg/L, VB₃ 1.5 mg/L, VB₁₂ 1.5 g/L, and pH adjusted to 7.0 with sodium hydroxide.

In order to evaluate the effect of the 5 pXMJ19-aglA vectors constructed in step I on isomaltose utilization, the 5 vectors are transformed into the wild-type Corynebacterium glutamicum ATCC13032 strain by electroporation (for the specific transformation method, see WO2014121669A1). Positive transformants are obtained by PCR identification using primers MJ19-F/MJ19-R, namely Corynebacterium glutamicum ATCC13032-pXMJ19-aglA-1, ATCC13032-pXMJ19-aglA-2, ATCC13032-pXMJ19-aglA-3, ATCC13032-pXMJ19-aglA-4, and ATCC13032-pXMJ19-aglA-5.

The above 5 positive transformants are cultured on culture plates containing chloramphenicol (34 mg/L) at 32°C for 12 h per generation, and after three consecutive passages, inoculated into 500 mL Erlenmeyer flasks containing 30 mL of rich medium, and subjected to shake flask fermentation at 30°C for 48 h. After the fermentation culture is completed, the concentration of isomaltose is detected by high-performance liquid chromatography (HPLC).

**Table 1: Isomaltose content detected by HPLC for ATCC13032-pXMJ19-aglA**

| Strain | Isomaltose content (g/L) |
|---|---|
| ATCC13032 | 4.8 |
| ATCC13032-pXMJ19-aglA-1 | 2.3 |
| ATCC13032-pXMJ19-aglA-2 | 1.6 |
| ATCC13032-pXMJ19-aglA-3 | 2.5 |
| ATCC13032-pXMJ19-aglA-4 | 1.4 |
| ATCC13032-pXMJ19-aglA-5 | 0.9 |

As shown in Table 1, all 5 Corynebacterium glutamicum ATCC13032-pXMJ19-aglA strains have the ability to degrade isomaltose, among which ATCC13032-pXMJ19-aglA-5 has the best isomaltose degradation ability, indicating that the aglA-5 gene has the highest isomaltose degradation activity.

### III. Construction of ATCC13869 strains containing aglA gene vectors

In order to evaluate the effect of the 5 pXMJ19-aglA vectors constructed in step I on isomaltose utilization, the 5 vectors are transformed into the wild-type Corynebacterium glutamicum ATCC13869 strain (CICC, catalog number 20216) by electroporation. Positive transformants ATCC13869-pXMJ19-aglA-1, ATCC13869-pXMJ19-aglA-2, ATCC13869-pXMJ19-aglA-3, ATCC13869-pXMJ19-aglA-4, and ATCC13869-pXMJ19-aglA-5 are obtained by PCR identification using primers MJ19-F/MJ19-R. The 5 positive transformants are inoculated on culture plates containing chloramphenicol (34 mg/L), cultured at 32°C for 12 h, and then single colonies are picked for streak culture. After three consecutive passages, the strains are inoculated into 500 mL Erlenmeyer flasks containing 30 mL of rich medium, and subjected to shake flask fermentation at 30°C for 48 h. After the fermentation culture is completed, the concentration of isomaltose is detected by HPLC.

**Table 2: Isomaltose content detected by HPLC for ATCC13869-pXMJ19-aglA**

| Strain | Isomaltose content (g/L) |
|---|---|
| ATCC13869 | 4.9 |
| ATCC13869-pXMJ19-aglA-1 | 3.1 |
| ATCC13869-pXMJ19-aglA-2 | 1.9 |
| ATCC13869-pXMJ19-aglA-3 | 2.7 |
| ATCC13869-pXMJ19-aglA-4 | 2.1 |
| ATCC13869-pXMJ19-aglA-5 | 1.2 |

As shown in Table 2, all 5 Corynebacterium glutamicum ATCC13869-pXMJ19-aglA strains have the ability to degrade isomaltose, among which ATCC13869-pXMJ19-aglA-5 has the best isomaltose degradation ability, indicating that the aglA-5 gene has the highest isomaltose degradation activity.

### IV. Construction of W3110 strains containing aglA gene vectors

In order to evaluate the effect of the 5 pXMJ19-aglA vectors constructed in step I on isomaltose utilization, the 5 vectors are transformed into the wild-type Escherichia coli W3110 strain (ATCC, catalog number 27325) by electroporation. Positive transformants W3110-pXMJ19-aglA-1, W3110-pXMJ19-aglA-2, W3110-pXMJ19-aglA-3, W3110-pXMJ19-aglA-4, and W3110-pXMJ19-aglA-5 are obtained by PCR identification using primers MJ19-F/MJ19-R. The 5 positive transformants are subjected to three consecutive passages on 2YT culture plates (chloramphenicol 34 mg/L), then inoculated into 500 mL Erlenmeyer flasks containing 30 mL of rich medium, and subjected to shake flask fermentation at 37°C for 24 h. After the fermentation culture is completed, the concentration of isomaltose is detected by HPLC.

**Table 3: Isomaltose content detected by HPLC for W3110-pXMJ19-aglA**

| Strain | Isomaltose content (g/L) |
|---|---|
| W3110 | 4.9 |
| W3110-pXMJ19-aglA-1 | 3.8 |
| W3110-pXMJ19-aglA-2 | 2.4 |
| W3110-pXMJ19-aglA-3 | 3.5 |
| W3110-pXMJ19-aglA-4 | 2.7 |
| W3110-pXMJ19-aglA-5 | 1 |

As shown in Table 3, all 5 Escherichia coli W3110-pXMJ19-aglA strains have the ability to degrade isomaltose, among which W3110-pXMJ19-aglA-5 has the best isomaltose degradation ability, indicating that the aglA-5 gene has the highest isomaltose degradation activity. Combined with the data from Tables 1, 2, and 3, it is demonstrated that the aglA-5 gene has the ability to degrade isomaltose in Corynebacterium glutamicum ATCC13032, ATCC13869, and Escherichia coli W3110, with the highest activity.

### Example 2: use of aglA-5 gene in the preparation of L-lysine-producing strains

### I. Construction of engineered strains expressing aglA-5 gene on a plasmid

The pXMJ19-aglA-5 plasmid successfully constructed in Example 1 is electroporated into wild-type Corynebacterium glutamicum ATCC13032 and Corynebacterium glutamicum YP097158 (L-lysine-producing strain, deposit number: CGMCC No.12856, deposit date: August 16, 2016, depositary: China General Microbiological Culture Collection Center, No.3, Yard 1, Beichen West Road, Chaoyang District, Beijing, telephone: 010-64807355), respectively, and cultured on culture plates containing chloramphenicol (34 mg/L) for 30 h. The resulting single colonies are identified by PCR using primers MJ19-F/MJ19-R (MJ19-F is located at 6570-6590 bp in the pXMJ19 vector, and MJ19-R is located at 69-87 bp in the pXMJ19 vector). Positive strains are those that amplified a fragment of 2014 bp (wherein nucleotides 1-141 are SEQ ID NO.45, nucleotides 142-1962 are the aglA-5 gene having the nucleotide sequence as shown in SEQ ID NO.6, and nucleotides 1963-2014 are SEQ ID NO.46).

The strains obtained using wild-type Corynebacterium glutamicum ATCC13032 and L-lysine-producing strain YP097158 as the starting strains are named YPL-aglA-1 and YPL-aglA-2, respectively. Recombinant strain YPL-aglA-1 contains a plasmid having the aglA gene shown in SEQ ID NO.6, which can significantly and stably increase the expression level of the aglA gene in wild-type Corynebacterium glutamicum ATCC13032. Recombinant strain YPL-aglA-2 contains a plasmid having the aglA gene shown in SEQ ID NO.6, which can significantly and stably increase the expression level of the aglA gene in L-lysine-producing strain YP097158.

### II. Construction of engineered strains expressing aglA-5 gene on the genome

Based on the Corynebacterium glutamicum ATCC13032 genome sequence published by NCBI, 3 pairs of primers for amplifying upstream and downstream homologous arm fragments and the coding region and promoter region of the aglA-5 gene are designed and synthesized, and a copy of the aglA-5 gene is inserted into L-lysine-producing strain YP097158 and wild-type Corynebacterium glutamicum ATCC13032 by homologous recombination.

The primers are designed as follows (synthesized by Shanghai Invitrogen Co., Ltd.):
P1:5'-CAGTGCCAAGCTTGCATGCCTGCAGGTCGACTCTAGAATGCGTTCTGGACTGA GG-3' (the underlined nucleotide sequence is the sequence on pK18, SEQ ID NO.13);
P2: 5'-CGTTTAGGGCACCAGATAGAgtgcaccgagaacagatg-3' (SEQ ID NO.14);
P3: 5'-catctgttctcggtgcacTCTATCTGGTGCCCTAAACG-3' (SEQ ID NO.15);
P4: 5'-cttgatttaattgcgccatctgTTACAGCTGCACTGCTGCTGCTTC-3' (SEQ ID NO.16);
P5: 5'-GAAGCAGCAGCAGTGCAGCTGTAAcagatggcgcaattaaatcaag-3' (SEQ ID NO.17);

P6:5'-CAGCTATGACCATGATTACGAATTCGAGCTCGGTACCCGCTATGACACCTTCAA
CGGATC-3' (SEQ ID NO.18).

In the above primers, the underlined nucleotide sequences are homologous sequences on the pK18mobsacB plasmid, used for integrating the DNA fragment (upstream homologous arm, promoter-aglA-5 gene, downstream homologous arm) into the pK18mobsacB plasmid (Addgene) by homologous recombination. The bases shown in lowercase letters on P2 and P3 are used for connecting the upstream homologous arm and the promoter-aglA-5 gene by homologous recombination, and the lowercase letters on P4 and P5 are used for connecting the promoter-aglA-5 gene and the downstream homologous arm by homologous recombination.

Construction method: using the Corynebacterium glutamicum ATCC13032 genome as a template, PCR amplification is performed using primers P1/P2 and P5/P6, respectively, to obtain an upstream homologous arm fragment of 763 bp (corresponding to positions 1928220 to 1928982 of the Corynebacterium glutamicum ATCC13032 genome sequence (GenBank: CP025533.1, 27-DEC-2017), i.e., SEQ ID NO.47) and a downstream homologous arm fragment of 596 bp (corresponding to positions 1929024 to 1929619 of the Corynebacterium glutamicum ATCC13032 genome sequence (GenBank: CP025533.1, 27-DEC-2017), i.e., SEQ ID NO.49). Using plasmid pXMJ19-aglA-5 as a template, PCR amplification is performed using primers P3/P4 to obtain the aglA-5 gene and its promoter fragment (i.e., promoter-aglA-5 gene) of 1901 bp (wherein nucleotides 1-80 are SEQ ID NO.48, and nucleotides 81-1901 are the aglA-5 gene (SEQ ID NO.6)). After the PCR reaction, the 3 amplified fragments are separately recovered by gel electrophoresis using a column-type DNA gel recovery kit. The 3 recovered fragments and the pK18mobsacB plasmid (Addgene) digested with XbalI and BamHI and purified are ligated using NEBuilder enzyme (NEB) at 50°C for 30 min. The single clones grown after transformation of the ligation products are identified by PCR using primers M13F (5'-TGTAAAACGACGGCCAGT-3', SEQ ID NO.19) / M13R (5'-CAGGAAACAGCTATGACC-3', SEQ ID NO.20) to obtain positive integration plasmids (recombinant vectors). The obtained recombinant vector is pK18-aglA(L). The positive integration plasmid contains a kanamycin resistance marker, and recombinants with the plasmid integrated into the genome can be obtained by kanamycin screening. The pK18-aglA(L) plasmid is a recombinant expression vector obtained by replacing the sequence between the XbalI and BamHI recognition sites of the pK18mobsacB plasmid with a DNA fragment while keeping other sequences unchanged. The DNA fragment is formed by homologous recombination in the 5'-3' direction of the upstream homologous arm (SEQ ID NO.47, 763 bp), the aglA-5 gene (SEQ ID NO.6) and its promoter fragment (SEQ ID NO.48, 80 bp), and the downstream homologous arm (partial coding region of the NCgl1742 gene, 596 bp).

PCR amplification system: 5× HiFi with Mg²⁺ Buffer 10 µL, dNTP Mixture (10 mM) 1.5 µL, each primer (10 pM) 1.6 µL, KAPA HiFi HotStart (1 U/µL) 0.5 µL, and ddH₂O added to a total volume of 50 µL.

PCR amplification program: pre-denaturation at 95°C for 5 min, (denaturation at 98°C for 20 s; annealing at 56°C for 15 s; extension at 72°C for 60 s; 30 cycles), and over-extension at 72°C for 5 min.

The correctly sequenced integration plasmid pK18-aglA(L) is electroporated into Corynebacterium glutamicum YP097158 and wild-type Corynebacterium glutamicum ATCC13032, respectively, and cultured on culture plates containing chloramphenicol (34 mg/L) for 30 h. The resulting single colonies are identified by PCR using primers P7/P8. Positive strains are those that amplified a fragment of 1488 bp (sequence of SEQ ID NO.50), and strains that did not amplify any fragment are the original strains. The positive strains are streaked on solid culture plates containing 15% sucrose and cultured for 30 h. The resulting single colonies are further identified by PCR using primers P9/P10. Strains that amplified a fragment of 1537 bp (sequence of SEQ ID NO.51) are positive strains in which the aglA-5 gene and its promoter are integrated into the intergenic region between the upstream homologous arm NCgl1741 and the downstream homologous arm NCgl1742 on the Corynebacterium glutamicum genome. The strains obtained using wild-type Corynebacterium glutamicum ATCC13032 and lysine-producing strain YP097158 as the starting strains are named YPL-aglA-3 and YPL-aglA-4, respectively.

Recombinant strains YPL-aglA-3 and YPL-aglA-4 contain the aglA gene shown in SEQ ID NO.6. Specifically, recombinant strain YPL-aglA-3 is obtained by replacing the intergenic region between the upstream homologous arm NCgl1741 and the downstream homologous arm NCgl1742 in the wild-type Corynebacterium glutamicum ATCC13032 genome with the aglA-5 gene and its promoter (formed by connecting SEQ ID NO.48 (80 bp) and SEQ ID NO.6 in the 5'-3' direction), i.e., replacing the nucleotide sequence at positions 1928983 to 1929023 of the Corynebacterium glutamicum ATCC13032 genome sequence (GenBank: CP025533.1, 27-DEC-2017) with a DNA fragment formed by connecting the H36 promoter sequence (SEQ ID NO.48, 80 bp) and SEQ ID NO.6 in the 5'-3' direction, while keeping other nucleotides in the Corynebacterium glutamicum ATCC13032 genome unchanged. Recombinant strain YPL-aglA-4 is obtained by replacing the intergenic region between the upstream homologous arm NCgl1741 and the downstream homologous arm NCgl1742 in the Corynebacterium glutamicum YP097158 genome (i.e., the nucleotide sequence corresponding to positions 1928983-1929023 of the Corynebacterium glutamicum ATCC13032 genome sequence (GenBank: CP025533.1, 27-DEC-2017)) with the aglA-5 gene and its promoter (a DNA fragment formed by connecting the H36 promoter sequence (SEQ ID NO.48, 80 bp) and SEQ ID NO.6 in the 5'-3' direction), while keeping other nucleotides in the Corynebacterium glutamicum YP097158 genome unchanged.

The PCR identification primers are as follows:
P7: 5'-TCCAAGGAAGATACACGCC-3' (corresponding to the outside of the upstream homologous arm NCgl1740, located at 1928088-1928106 bp of the Corynebacterium glutamicum ATCC13032 genome, SEQ ID NO.21);
P8: 5'-GCTACCACCAAAGTAAGAGCC-3' (corresponding to the inside of the aglA gene, located at 493-513 bp of the coding region, SEQ ID NO.22);
P9: 5'-TTGTGATGGCGTGAAGTG-3' (corresponding to the inside of the aglA gene, located at 972-989 bp of the coding region, SEQ ID NO.23); and
P10: 5'-TGGTCGTTGGAATCTTGC-3' (corresponding to the outside of the downstream homologous arm NCgl1742, located at 1929693-1929710 bp of the Corynebacterium glutamicum ATCC13032 genome, SEQ ID NO.24).

### III. L-lysine fermentation experiment

YPL-aglA-1 and YPL-aglA-2 constructed in step I, YPL-aglA-3 and YPL-aglA-4 constructed in step II, L-lysine-producing strain YP097158, and wild-type Corynebacterium glutamicum ATCC13032 are subjected to fermentation experiments in a BLBIO-5GC-4-H type fermenter (Shanghai Bailun Biotechnology Co., Ltd.) using the following medium and L-lysine fermentation process. After fermentation, the L-lysine content is detected using an SBA biosensor (purchased from Shandong Academy of Sciences, Institute of Biology). Each strain is repeated three times, and the results are shown in Table 4. Fermentation medium composition (the rest is water): starch hydrolyzate 30 g/L, ammonium sulfate 12 g/L, magnesium sulfate 0.87 g/L, molasses 20 g/L, acidified corn steep liquor 3 mL/L, phosphoric acid 0.4 mL/L, potassium chloride 0.53 g/L, antifoaming agent (2% polydimethylsiloxane) 4 mL/L, ferrous sulfate 120 mg/L, manganese sulfate 120 mg/L, nicotinamide 42 mg/L, calcium pantothenate 6.3 mg/L, vitamin B1 6.3 mg/L, copper and zinc salt solution 0.6 g/L, biotin 0.88 mg/L.

L-lysine fermentation process: fermentation conditions: temperature 37°C, time 48 h, pH 6.9 ± 0.05, dissolved oxygen DO 10-30%, inoculum size 10% (v/v); initial control conditions: temperature 37°C, pH 6.9, tank pressure 0 MPa, airflow rate 3 L/min, agitation speed 550 rpm; dissolved oxygen control: when DO < 30%, sequentially increase agitation speed to 750 rpm → 800 rpm → 850 rpm → 950 rpm, airflow rate 4 L/min; tank pressure control: increase tank pressure to 0.01 MPa at 6 h of fermentation; at 12 h, increase tank pressure to 0.02 MPa → 0.03 MPa → 0.04 MPa → 0.05 MPa; residual sugar control: 0.1-0.2% before 12 h of fermentation; after 12 h of fermentation, control residual sugar at 0.1-0.05% in combination with DO requirements; ammonia nitrogen control: 0.1-0.15 before 12 h of fermentation; 0.15-0.25 from 12 h to 32 h of fermentation; 0.1-0.15 after 32 h of fermentation; feed materials: 25% ammonia water, 70% concentrated sugar, 50% ammonium sulfate, 10% antifoaming agent (polydimethylsiloxane).

**Table 4: L-lysine fermentation data of aglA engineered strains**

| | L-lysine concentration (g/L) | | Mean (g/L) | | Significance |
|---|---|---|---|---|---|
| Strain | Ferment ation 1 | Fermenta tion 2 | Fermentation 3 | | analysis |
| ATCC13032 | 0.009 | 0.013 | 0.007 | 0.010 | |
| YPL-aglA-1 | 0.522 | 0.492 | 0.613 | 0.542 | P<0.01 |
| YPL-aglA-3 | 0.322 | 0.248 | 0.413 | 0.328 | P<0.01 |

| Strain | L-lysine concentration (g/100mL) | | | Mean (g/100mL) | Significance analysis |
|---|---|---|---|---|---|
| | Ferment ation 1 | Fermenta tion 2 | Fermentation 3 | | |
| YP097158 | 18.97 | 18.46 | 18.34 | 18.59 | |
| YPL-aglA-2 | 20.54 | 21.38 | 20.87 | 20.93 | P<0.01 |
| YPL-aglA-4 | 20.12 | 19.93 | 19.65 | 19.9 | P<0.01 |

As shown in Table 4, overexpression of the aglA gene in both wild-type Corynebacterium glutamicum ATCC13032 and L-lysine-producing strain YP097158 contributes to an increase in L-lysine yield.

### Example 3: use of aglA-5 Gene in the Preparation of L-Threonine-Producing Strains

### I. Construction of Engineered Strains Expressing aglA-5 Gene on Plasmids

2YT Agar Plate Recipe: Yeast extract 10 g/L, peptone 16 g/L, NaCl 5 g/L, agar 18 g/L, pH adjusted to 7.0 with NaOH, autoclaved at 121°C for 15 min.

The pXMJ19-aglA-5 plasmid successfully constructed in Example 1 is electroporated into wild-type Escherichia coli W3110 (ATCC, Cat. No. 27325) and E. coli CGMCC No. 25404 (i.e., Escherichia coli, Deposit No.: CGMCC No. 25404, Deposit Date: July 25, 2022, Depository: China General Microbiological Culture Collection Center, No. 1 Beichen West Road, Chaoyang District, Beijing, Tel: 010-64807288), respectively. The transformants are cultured on 2YT agar plates for 18 h. Single colonies are subjected to PCR identification using primers MJ19-F/MJ19-R. Strains yielding a 2014 bp fragment (wherein nucleotides 1-141 correspond to SEQ ID No. 45, nucleotides 142-1962 correspond to the aglA-5 gene having the nucleotide sequence shown in SEQ ID No. 6, and nucleotides 1963-2014 correspond to SEQ ID No. 46) are identified as positive strains. The strains derived from wild-type E. coli W3110 and the L-threonine-producing strain CGMCC25404 are designated YPT-aglA-1 and YPT-aglA-2, respectively. Recombinant strain YPT-aglA-1 harbors a plasmid containing the aglA gene shown in SEQ ID No. 6, which can significantly and stably enhance the expression level of the aglA gene in wild-type E. coli W3110. Recombinant strain YPT-aglA-2 harbors a plasmid containing the aglA gene shown in SEQ ID No. 6, which can significantly and stably enhance the expression level of the aglA gene in the L-threonine-producing strain CGMCC25404.

### II. Construction of Engineered Strains Expressing aglA-5 Gene on the Genome

Based on the E. coli W3110 genome sequence published by NCBI, the CRISPR/Cas9 gene editing technology is employed to integrate the aglA-5 gene into the yaiT coding region of the L-threonine-producing strain CGMCC25404 and wild-type E. coli W3110, in order to further investigate the effect of the aglA-5 gene on L-threonine production.

### (1) Construction of sgRNA

Based on the E. coli W3110 genome sequence published by NCBI (GenBank accession no. AP009048.1), sgRNA target sequences are designed using CRISPR RGEN Tools (http://www.rgenome.net/cas-designer/). After selecting appropriate sgRNA target sequences, the terminal sequences of the linearized pGRB cloning vector are added to the 5' and 3' ends of the target sequences to facilitate the formation of a complete sgRNA plasmid via recombination.

The sgRNA fragment is amplified without a template; only a PCR annealing process is required. The reaction system and program are as follows: PCR reaction system: sgRNA-2F 10 µL, sgRNA-2R 10 µL. PCR reaction program: 95°C denaturation for 5 min, 50°C annealing for 1 min. After annealing, the target fragment is recovered using a DNA purification kit, and its DNA concentration is measured and diluted to 100 ng/µL.

The pGRB plasmid is extracted and digested with Spe I followed by dephosphorylation to prevent self-ligation of the pGRB plasmid. Digestion system: 10× Buffer 5 µL, Spe 12.5 µL, pGRB plasmid DNA 3000-5000 ng, ddH₂O to 50 µL. After incubation at 37°C for 3 h, the linearized fragment is recovered by agarose gel electrophoresis and gel extraction, then subjected to dephosphorylation. Dephosphorylation system: 10× Buffer 5 µL, pGRB plasmid DNA 1000-2000 ng, CIAP 2.5 µL, ddH₂O to 50 µL. After incubation at 37°C for 1 h, the linearized pGRB plasmid is recovered using a DNA purification kit. Recombination of the sgRNA fragment with the pGRB plasmid is then performed using a Gibson Assembly Kit (New England Biolabs). Recombination system: NEB Assembly Enzyme 2.5 µL, linearized cloning vector 2 µL, sgRNA 0.5 µL. After assembly at 50°C for 30 min, the product is transformed into DH5α competent cells. Plasmids are extracted and verified by sequencing using primers sgRNA-PF/sgRNA-PR. The plasmid with correct sequence is designated pGRB-sgRNA-1.

The primers used in this experiment are designed as follows (synthesized by Shanghai Invitrogen Co., Ltd.). Bases underlined represent the pGRB cloning vector homology arm sequences, and bases in lowercase represent the sgRNA sequence, which targets nucleotides 389640 to 389659 of the W3110 genome sequence (GenBank accession no. AP009048.1):
sgRNA-2F: 5′-TGACAGCTAGCTCAGTCCTAGGTATAATACTAGTggcaactatgtaaactatagGTTTTAGAGCTA GAAATAGCAAGTTAAAATAAGG-3′ (SEQ ID No. 25)
sgRNA-2R: 
5′-CCTTATTTTAACTTGCTATTTCTAGCTCTAAAACctatagtttacatagttgccACTAGTATTATACCT
AGGACTGAGCTAGCTGTCA-3′ (SEQ ID No. 26)
sgRNA-PF: 5'-GTCTCATGAGCGGATACATATTTG-3' (SEQ ID No. 27)
sgRNA-PR: 5'-ACTGGCACTGCTGTGCGCCG-3' (SEQ ID No. 28)

### (2) PCR Amplification of Homologous DNA Fragments

Based on the E. coli W3110 genome sequence published by NCBI, three pairs of primers are designed and synthesized to amplify the upstream and downstream homology arm sequences as well as the coding and promoter regions of the aglA-5 gene. The aglA-5 gene is introduced into the yaiT coding region of the L-threonine-producing strain CGMCC25404 and E. coli W3110, respectively, via CRISPR/Cas9 gene editing.

The primers are designed as follows (synthesized by Shanghai Invitrogen Co., Ltd.):
P11: 5'-AAGAGAATGGAAGAGAGGCC-3' (SEQ ID No.29) ;
P12: 5'- CGTTTAGGGCACCAGATAGAcccaatcaagtgctgtaacg -3' (SEQ ID No.30) ;
P13: 5'- cgttacagcacttgattgggTCTATCTGGTGCCCTAAACG -3' (SEQ ID No.31 ) ;
P14: 5'- cggtagtgtaggtttcgttgTTACAGCTGCACTGCTGCTGCTTC-3' (SEQ ID No.32 ) ;
P15: 5'- GAAGCAGCAGCAGTGCAGCTGTAAcaacgaaacctacactaccg -3' (SEQ ID No.33 ) ;
P16: 5'-CGACCTGTAGTATCCCATTC-3' (SEQ ID No.34) _{∘}

Using W3110 genomic DNA as the template, PCR amplification is performed with primer pairs P11/P12 and P15/P16, respectively, using KAPA HiFi HotStart (KAPA, Catalog No. KR1501) as the enzyme, to obtain the upstream homologous arm fragment of 570 bp (SEQ ID No. 52, corresponding to nucleotides 389041-389610 of the W3110 genome) and the downstream homologous arm fragment of 585 bp (SEQ ID No. 53, corresponding to nucleotides 390148-390733 of the W3110 genome). Using plasmid pXMJ19-aglA-5 as the template, PCR amplification is performed with primer pair P13/P14 to obtain the aglA-5 gene and its promoter fragment of 1901 bp (wherein nucleotides 1-80 correspond to the H36 promoter (80 bp), and nucleotides 81-1901 correspond to the aglA-5 gene (SEQ ID No. 6)). After the PCR reactions are completed, the three DNA fragments are recovered from agarose gel electrophoresis using a column-type DNA gel recovery kit, respectively. The three recovered DNA fragments are then subjected to overlap PCR using primers P11 and P16 to obtain the genomic overexpression DNA recombination fragment Up-aglA-Down (whose sequence is composed of SEQ ID No. 52, SEQ ID No. 48, SEQ ID No. 6 (1821 bp), and SEQ ID No. 53 connected in the 5'-3' direction), with a size of 3056 bp.

PCR Amplification System: 5× HiFi with Mg²⁺ Buffer 10 µL, dNTP Mixture (10 mM) 1.5 µL, each primer (10 pM) 1.6 µL, KAPA HiFi HotStart (1 U/µL) 0.5 µL, ddH₂O added to a total volume of 50 µL.

PCR Amplification Program: 95°C pre-denaturation for 5 min, (98°C denaturation for 20 s; 56°C annealing for 15 s; 72°C extension for 60 s; 30 cycles), 72°C final extension for 5 min.

### (3) Preparation of Competent Cells and Transformation

2-YT Agar Plates Containing Spectinomycin (100 mg/L): Yeast extract 10 g/L, peptone 16 g/L, NaCl 5 g/L, agar 18 g/L, spectinomycin 100 mg/L. The pH is adjusted to 7.0 with NaOH, followed by autoclaving at 121°C for 15 min.

The pREDCas9 plasmid (containing a spectinomycin resistance gene) (Addgene, Catalog No. 71541) is extracted and transformed into competent cells of the L-threonine-producing strain CGMCC25404 and E. coli W3110, respectively. The transformants are plated on 2-YT agar plates containing spectinomycin (100 mg/L) and incubated at 32°C. Spectinomycin-resistant (100 mg/L) single colonies are picked and verified by PCR using primers pRedCas9-PF (5'-GCAGTGGCGGTTTTCATG-3', SEQ ID No. 35) / pRedCas9-PR (5'-CCTTGGTGATCTCGCCTTTC-3', SEQ ID No. 36). Colonies yielding the fragment of SEQ ID No. 54 are identified as CGMCC25404-Cas9 and W3110-Cas9 transformants harboring the pREDCas9 plasmid.

Competent cells of the L-threonine-producing strain CGMCC25404-Cas9 and W3110-Cas9 are prepared. When the culture reached OD₆₀₀ₙₘ = 0.1, IPTG is added to a final concentration of 0.1 mM to induce λ-Red-mediated homologous recombination. When OD₆₀₀ₙₘ = 0.4, cells are collected and made competent, then co-transformed with the pGRB-sgRNA-1 plasmid and the genomic recombination fragment Up-aglA-Down. The transformants are plated on 2-YT agar plates containing spectinomycin (100 mg/L) and ampicillin (100 mg/L) and incubated at 32°C for 12 h. Single colonies are subcultured and verified by PCR using primers P11/P16. Colonies yielding a 3056 bp fragment (whose sequence is composed of SEQ ID No. 52 (570 bp), SEQ ID No. 48 (80 bp), SEQ ID No. 6 (1821 bp), and SEQ ID No. 53 (585 bp) connected in the 5'-3' direction) are identified as positive transformants.

PCR Amplification System: 2× Premix r Taq 12.5 µL, each primer (10 pM) 1 µL, ddH₂O added to a total volume of 25 µL.PCR Amplification Program: 94°C pre-denaturation for 5 min, 94°C denaturation for 30 s; 56°C annealing for 30 s; 72°C extension for 3 min (30 cycles), 72°C final extension for 10 min.

Positive transformants are inoculated into 2-YT medium containing spectinomycin (100 mg/L) and L-arabinose at a final concentration of 0.2% (w/v) to eliminate the pGRB-sgRNA-1 plasmid. Colonies that grew on spectinomycin (100 mg/L) plates but not on ampicillin (100 mg/L) plates are selected, then transferred to 2-YT medium and incubated at 42°C to eliminate the pREDCas9 plasmid. Colonies that did not grow on spectinomycin (100 mg/L) plates but grew on antibiotic-free 2-YT medium are selected and again verified by PCR using primers P11/P16. Clones capable of amplifying the 3056 bp fragment are confirmed as positive and sent for sequencing. The strains with correct sequencing results are designated YPT-aglA-3 (derived from E. coli W3110) and YPT-aglA-4 (derived from the L-threonine-producing strain CGMCC25404), respectively.

Recombinant strains YPT-aglA-3 and YPT-aglA-4 contain the aglA-5 gene shown in SEQ ID No. 6. Specifically, recombinant strain YPT-aglA-3 is constructed by replacing a partial coding region of the yaiT gene (i.e., nucleotides 389611-390147 of the W3110 genome sequence, GenBank Accession No. AP009048.1) on the E. coli W3110 chromosome with the aglA-5 gene and its promoter (wherein nucleotides 1-80 correspond to the H36 promoter (SEQ ID No. 48, 80 bp), and nucleotides 81-1901 correspond to the aglA-5 gene (SEQ ID No. 6)), while keeping all other nucleotides in the genome unchanged. Recombinant strain YPT-aglA-4 is constructed by replacing the corresponding partial yaiT coding region (corresponding to nucleotides 389611-390147 of the W3110 genome sequence, GenBank Accession No. AP009048.1) on the chromosome of the L-threonine-producing strain CGMCC25404 with the aglA-5 gene and its promoter (wherein nucleotides 1-80 correspond to the H36 promoter (SEQ ID No. 48, 80 bp), and nucleotides 81-1901 correspond to the aglA-5 gene (SEQ ID No. 6)), while keeping all other nucleotides in the genome unchanged. The recombinant strains harboring the aglA-5 gene can significantly and stably enhance the expression level of the aglA gene.

### III. L-Threonine Fermentation Experiments

The strains YPT-aglA-1 and YPT-aglA-2 constructed in Step I, the strains YPT-aglA-3 and YPT-aglA-4 constructed in Step II, wild-type E. coli W3110, and the L-threonine-producing strain CGMCC25404 are each inoculated into a 5 L fermenter (model BLBIO-5GC-4-H, Shanghai Bailun Biotechnology Co., Ltd.) for fermentation experiments using L-threonine fermentation medium under the specified culture conditions. Each strain is tested in triplicate.

L-Threonine Fermentation Medium: The solvent is water. The solutes and their concentrations are: glucose 13 g/L, (NH4)₂SO₄ 1 g/L, H₃PO₄ 0.5 g/L, KCl 0.8 g/L, MgSO₄·7H₂O 0.8 g/L, FeSO₄·7H₂O 0.01 g/L, MnSO₄·H₂O 0.01 g/L, ZnSO₄ 0.05 g/L, FM902 yeast powder 1.5 g/L, corn steep liquor 5 g/L, molasses 17 g/L. The pH is adjusted to 7.0 with ammonia.

L-Threonine Fermentation Conditions: Calibration: DO corrected to 100%; temperature 37°C, airflow 5 L/min, agitation 800 rpm, tank pressure 0 MPa; calibrated after 5 min. Inoculum: 10%.Initial conditions: pH 7.0, temperature 37°C, tank pressure 0 MPa, airflow 0.5 L/min, agitation 400 rpm. Process control: 1.When DO < 30%, sequentially increase agitation: 500 rpm → 600 rpm → airflow 1 L/min → 700 rpm → 800 rpm.2.At 8 h, increase tank pressure to 0.01 MPa; at 12 h, increase tank pressure: 0.02 MPa → 0.03 MPa → 0.04 MPa → 0.05 MPa.Residual sugar control: Before 12 h, maintain at 0.1-0.5%; after 12 h, maintain at 0.1-0.3% in conjunction with DO requirements. Feed materials: 25% ammonia water, 55% concentrated sugar solution, 10% antifoam. Fermentation duration: approximately 30 h, with airflow adjusted up or down based on DO maintained at 20-30%.

**Table 5: L-Threonine Fermentation Data of aglA Engineered Strains**

| Strain | L-Threonine Concentration (g/L) | | | | Significance Analysis |
|---|---|---|---|---|---|
| | Fermenta tion 1 | Ferment ation 2 | Ferment ation 3 | Average | |
| W3110 | 0.05 | 0.03 | 0.07 | 0.05 | |
| YPT-aglA-1 | 0.83 | 0.78 | 0.45 | 0.69 | P<0.01 |
| YPT-aglA-3 | 0.34 | 0.37 | 0.26 | 0.32 | P<0.01 |
| CGMCC25404 | 98.37 | 99.13 | 98.74 | 98.75 | |
| YPT-aglA-2 | 103.82 | 104.47 | 104.15 | 104.15 | P < 0.01 |
| YPT-aglA-4 | 102.58 | 101.84 | 102.36 | 102.26 | P < 0.01 |

As shown in Table 5, overexpression of the aglA-5 gene in both wild-type E. coli W3110 and the L-threonine-producing strain CGMCC25404 contributed to an increase in L-threonine production.

### Example 4: use of aglA-5 Gene in the Preparation of L-Glutamic Acid-Producing Strains

### I. Construction of Engineered Strains Expressing aglA-5 Gene on Plasmids

The pXMJ19-aglA-5 plasmid successfully constructed in Example 1 is electroporated into wild-type Corynebacterium glutamicum ATCC13869 and Corynebacterium glutamicum CGMCC No. 21220 (L-glutamic acid-producing strain, Deposit No.: CGMCC No. 21220, Deposit Date: November 23, 2020, Depository: China General Microbiological Culture Collection Center, No. 3, Yard 1, Beichen West Road, Chaoyang District, Beijing, Tel: 010-64807288), respectively, and cultured on agar plates containing chloramphenicol (34 mg/L) for 30 h. Single colonies are subjected to PCR identification using primers MJ19-F/MJ19-R. Strains yielding a 2014 bp fragment (wherein nucleotides 1-141 correspond to SEQ ID No. 45, nucleotides 142-1962 correspond to the aglA-5 gene having the nucleotide sequence shown in SEQ ID No. 6, and nucleotides 1963-2014 correspond to SEQ ID No. 46) are identified as positive strains. The strains derived from wild-type Corynebacterium glutamicum ATCC13869 and the L-glutamic acid-producing strain CGMCC No. 21220 are designated YPG-aglA-1 and YPG-aglA-2, respectively.

Recombinant strain YPG-aglA-1 harbors a plasmid containing the aglA gene shown in SEQ ID No. 6, which can significantly and stably enhance the expression level of the aglA gene in wild-type Corynebacterium glutamicum ATCC13869. Recombinant strain YPG-aglA-2 harbors a plasmid containing the aglA gene shown in SEQ ID No. 6, which can significantly and stably enhance the expression level of the aglA gene in the L-glutamic acid-producing strain CGMCC No. 21220.

### II. Construction of Engineered Strains Expressing aglA-5 Gene on the Genome

Based on the Corynebacterium glutamicum ATCC13869 genome sequence published by NCBI, three pairs of primers are designed and synthesized to amplify the upstream and downstream homology arm fragments as well as the coding and promoter regions of the aglA-5 gene. A copy of the aglA-5 gene is inserted into the L-glutamic acid-producing strain CGMCC No. 21220 and wild-type Corynebacterium glutamicum ATCC13869 by homologous recombination.

The primers are designed as follows (synthesized by Shanghai Invitrogen Co., Ltd.):
P17: 
5′-CAGTGCCAAGCTTGCATGCCTGCAGGTCGACTCTAGGACCCGCTTGCCATACGAAG-3′
(SEQ ID No. 37)
P18: 5'-CGTTTAGGGCACCAGATAGAatctactcatctgaagaatc-3' (SEQ ID No. 38)
P19: 5'-gattcttcagatgagtagatTCTATCTGGTGCCCTAAACG-3' (SEQ ID No. 39)
P20: 5'-caaaccagagtgcccacgaaTTACAGCTGCACTGCTGCTGCTTC-3' (SEQ ID No. 40)
P21: 5'-GAAGCAGCAGCAGTGCAGCTGTAAttcgtgggcactctggtttg-3' (SEQ ID No. 41)
P22:

5′-CAGCTATGACCATGATTACGAATTCGAGCTCGGTACCCGCATAAGAAACAACCACTTC
C-3′ (SEQ ID No. 42)

In the above primers, the underlined nucleotide sequences are homology sequences on the pK18 plasmid, used for integrating the DNA fragment (upstream homology arm, promoter-aglA-5 gene, downstream homology arm) into the pK18mobsacB plasmid (Addgene) by homologous recombination. The bases shown in lowercase letters on P18 and P19 are used for connecting the upstream homology arm and the promoter-aglA-5 gene by homologous recombination, and the lowercase letters on P20 and P21 are used for connecting the promoter-aglA-5 gene and the downstream homology arm by homologous recombination.

Construction method: Using the Corynebacterium glutamicum CGMCC No. 21220 genome as a template, PCR amplification is performed using primers P17/P18 and P21/P22, respectively, to obtain an upstream homology arm fragment of 750 bp (corresponding to positions 731131 to 731880 of the Corynebacterium glutamicum ATCC13869 genome sequence (GenBank accession no. CP016335.1), i.e., the BBD29_03485 gene, with the sequence of SEQ ID No. 55) and a downstream homology arm fragment of 731 bp (corresponding to positions 732071 to 732801 of the Corynebacterium glutamicum ATCC13869 genome sequence (GenBank accession no. CP016335.1), i.e., the BBD29_03490 gene, with the sequence of SEQ ID No. 56). Using plasmid pXMJ19-aglA-5 as a template, PCR amplification is performed using primers P19/P20 to obtain the aglA-5 gene and its promoter fragment (i.e., promoter-aglA-5 gene) of 1901 bp (wherein nucleotides 1-80 correspond to the H36 promoter (SEQ ID No. 48, 80 bp) and nucleotides 81-1901 correspond to the aglA-5 gene (SEQ ID No. 6)). After the PCR reaction, the three amplified fragments are separately recovered by gel electrophoresis using a column-type DNA gel recovery kit. The three recovered fragments and the pK18mobsacB plasmid (Addgene) digested with XbalI and BamHI and purified are ligated using NEBuilder enzyme (NEB) at 50°C for 30 min. Single clones grown after transformation of the ligation products are identified by PCR using primers M13F (5'-TGTAAAACGACGGCCAGT-3', SEQ ID No. 19) / M13R (5'-CAGGAAACAGCTATGACC-3', SEQ ID No. 20) to obtain positive integration plasmids (recombinant vectors). The obtained recombinant vector is designated pK18-aglA(G). The positive integration plasmid contains a kanamycin resistance marker, and recombinants with the plasmid integrated into the genome can be obtained by kanamycin screening. The pK18-aglA(G) plasmid is a recombinant expression vector obtained by replacing the sequence between the XbalI and BamHI recognition sites of the pK18mobsacB plasmid with a DNA fragment while keeping other sequences unchanged. The DNA fragment is formed by homologous recombination in the 5'→3' direction of SEQ ID No. 55 (750 bp), the aglA-5 gene and its promoter fragment (1901 bp), and SEQ ID No. 56 (731 bp).

PCR amplification system: 5× HiFi with Mg²⁺ Buffer 10 µL, dNTP Mixture (10 mM) 1.5 µL, each primer (10 pM) 1.6 µL, KAPA HiFi HotStart (1 U/µL) 0.5 µL, and ddH₂O added to a total volume of 50 µL. PCR amplification program: pre-denaturation at 95°C for 5 min, (denaturation at 98°C for 20 s; annealing at 56°C for 15 s; extension at 72°C for 60 s; 30 cycles), and over-extension at 72°C for 5 min.

The correctly sequenced integration plasmid pK18-aglA(G) is electroporated into Corynebacterium glutamicum CGMCC No. 21220 and wild-type Corynebacterium glutamicum ATCC13869, respectively, and cultured on agar plates containing chloramphenicol (34 mg/L) for 30 h. Single colonies are subjected to PCR identification using primers P23/P8. Positive strains are those that amplified a 1423 bp fragment (the fragment amplified by identification primers P23 and P8), and strains that did not amplify any fragment are the original strains. Positive strains are streaked on solid agar plates containing 15% sucrose and cultured for 30 h. Single colonies are further identified by PCR using primers P9/P24. Strains that amplified a fragment of 1640 bp (sequence as shown in SEQ ID No. 58) are positive strains in which the aglA-5 gene and its promoter are integrated into the intergenic region between the upstream homology arm BBD29_03485 and the downstream homology arm BBD29_03490 on the Corynebacterium glutamicum genome. The strains derived from wild-type Corynebacterium glutamicum ATCC13869 and the L-glutamic acid-producing strain CGMCC No. 21220 are designated YPG-aglA-3 and YPG-aglA-4, respectively.

Recombinant strains YPG-aglA-3 and YPG-aglA-4 contain the aglA gene shown in SEQ ID No. 6. Specifically, recombinant strain YPG-aglA-3 is constructed by replacing the intergenic region between the upstream homology arm BBD29_03485 and the downstream homology arm BBD29_03490 in the wild-type Corynebacterium glutamicum ATCC13869 genome with the aglA-5 gene and its promoter (wherein nucleotides 1-80 correspond to the H36 promoter (SEQ ID No. 48, 80 bp) and nucleotides 81-1901 correspond to the aglA-5 gene (SEQ ID No. 8)), i.e., replacing the nucleotide sequence at positions 731881 to 732070 of the Corynebacterium glutamicum ATCC13869 genome sequence (GenBank accession no. CP016335.1) with a DNA molecule formed by connecting SEQ ID No. 48 and SEQ ID No. 6 in the 5'-3' direction, while the remaining nucleotides in the Corynebacterium glutamicum ATCC13869 genome are kept unchanged. Recombinant strain YPG-aglA-4 is constructed by replacing the intergenic region between the upstream homology arm BBD29_03485 and the downstream homology arm BBD29_03490 in the Corynebacterium glutamicum CGMCC No. 21220 genome (corresponding to positions 731881 to 732070 of the Corynebacterium glutamicum ATCC13869 genome sequence) with the aglA-5 gene and its promoter (wherein nucleotides 1-80 correspond to the H36 promoter (SEQ ID No. 48, 80 bp) and nucleotides 81-1901 correspond to the aglA-5 gene (SEQ ID No. 8)), while the remaining nucleotides in the Corynebacterium glutamicum CGMCC No. 21220 genome are kept unchanged. Recombinant strains overexpressing the aglA-5 gene on the genome can significantly and stably enhance the expression level of the aglA gene.

PCR identification primers are as follows:
P23: 5'-GTCCAAGGTGACGGCCGCAC-3' (corresponding to the outside of the upstream homology arm BBD29_03485, SEQ ID No. 43)
P8: 5'-GCTACCACCAAAGTAAGAGCC-3' (corresponding to the inside of the aglA gene, SEQ ID No. 22)
P9: 5'-TTGTGATGGCGTGAAGTG-3' (corresponding to the inside of the aglA gene, SEQ ID No. 23)
P24: 5'-ATATTCGGCCCAGCAGCAGC-3' (corresponding to the outside of the downstream homology arm BBD29_03490, SEQ ID No. 44)

### III. L-Glutamic Acid Fermentation Experiment

The strains YPG-aglA-1 and YPG-aglA-2 constructed in Step I, the strains YPG-aglA-3 and YPG-aglA-4 constructed in Step II, wild-type Corynebacterium glutamicum ATCC13869, and the L-glutamic acid-producing strain CGMCC No. 21220 are each inoculated into a fermenter (model BLBIO-5GC-4-H, purchased from Shanghai Bailun Biotechnology Co., Ltd.) for fermentation experiments using the medium and the control process shown in Table 6 below. Each strain is tested in triplicate. Fermentation medium composition (the rest is water): glucose 5.0 g/L, phosphoric acid 0.38 g/L, magnesium sulfate 1.85 g/L, potassium chloride 1.6 g/L, biotin 550 µg/L, vitamin B1 300 µg/L, ferrous sulfate 10 mg/L, manganese sulfate 10 g/dL, KH2PO4 2.8 g/L, vitamin C 0.75 mg/L, vitamin B12 2.5 µg/L, p-aminobenzoic acid 0.75 mg/L, antifoaming agent 0.0015 mL/dL, betaine 1.5 g/L, cane molasses 7 mL/L, corn steep liquor 77 mL/L, aspartic acid 1.7 g/L, hair powder 2 g/L.

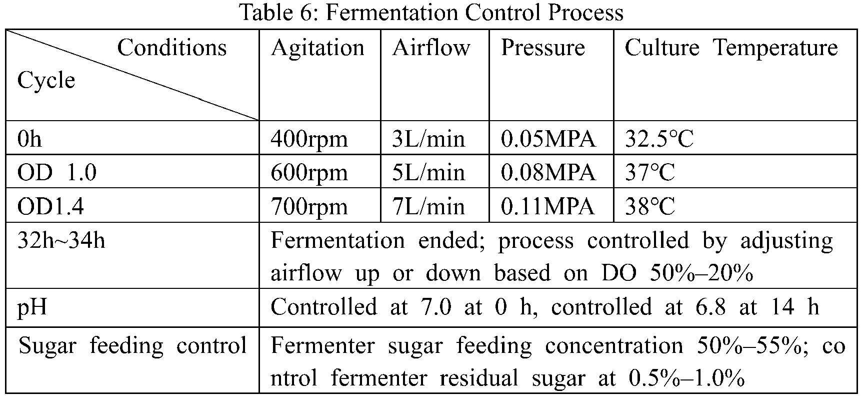

**Table 7: L-Glutamic Acid Fermentation Data of aglA Engineered Strains**

| Strain | L-Glutamic Acid Concentration (g/L) | | | | Significance Analysis |
|---|---|---|---|---|---|
| | Ferment ation 1 | Ferme ntation 2 | Ferment ation 3 | Average | |
| ATCC13869 | 0.02 | 0.05 | 0.04 | 0.04 | |
| YPG-aglA-1 | 0.84 | 0.77 | 0.63 | 0.75 | P<0.01 |
| YPG-aglA-3 | 0.49 | 0.58 | *0.55* | 0.54 | P<0.01 |
| CGMCC No.21220 | 100.76 | 101.24 | 101.43 | 101.14 | - |
| YPG-aglA-2 | 106.54 | 105.89 | 106.37 | 106.27 | P<0.01 |
| YPG-aglA-4 | 105.82 | 105.11 | 105.48 | 105.47 | P<0.01 |

As shown in Table 7, overexpression of the aglA gene in both wild-type Corynebacterium glutamicum ATCC13869 and the L-glutamic acid-producing strain CGMCC No. 21220 contributes to an increase in L-glutamic acid production.

The present application has been described in detail above. For those skilled in the art, without departing from the spirit and scope of the present application and without the need for unnecessary experiments, the present application can be practiced within a broad range under equivalent parameters, concentrations, and conditions. Although specific examples have been provided in the present application, it should be understood that further improvements can be made to the present application. In summary, in accordance with the principles of the present application, the present application is intended to comprise any modifications, uses, or improvements to the present application, including changes made using conventional techniques known in the art that depart from the scope already disclosed in the present application.

### Cross-reference to related applications:

The present application claims priority to the Chinese patent application filed with the China National Intellectual Property Administration on December 8, 2023, with application number 202311679969.1, and entitled "Application of aglA Gene in the Preparation of L-Amino Acids", the entire contents of which are hereby incorporated by reference.

### Industrial Applicability

The present application can significantly increase the yield of L-amino acids.

## Claims

1. A use, wherein, the use comprises any one of the following:
U1) use of α-glucosidase in the preparation of L-amino acids;
U2) use of a substance that regulates the activity of the α-glucosidase in the preparation of L-amino acids; and
U3) use of a substance that regulates the content of the α-glucosidase in the preparation of L-amino acids.

2. The use according to claim 1, wherein, the regulation comprises upregulating or enhancing or increasing the expression level of the α-glucosidase.

3. The use according to claim 2, wherein, the α-glucosidase comprises any one of the following:
A1) a protein having the amino acid sequence of SEQ ID No.5;
A2) a protein derived from A1) or having more than 45% identity with the protein shown in A1) and having the same function, obtained by substitution and/or deletion and/or addition of amino acid residues to the amino acid sequence shown in SEQ ID No.5;
or A3) a fusion protein having the same function obtained by linking a tag to the N-terminus and/or C-terminus of a1) or a2).

4. The use according to claim 3, wherein, the L-amino acid comprises polar neutral amino acids, acidic amino acids, basic amino acids, and/or nonpolar hydrophobic amino acids.

5. The use according to claim 4, wherein, the basic amino acids comprise lysine, arginine, and/or histidine, or the polar neutral amino acids comprise tryptophan, tyrosine, serine, threonine, cysteine, methionine, glutamine, or asparagine.

6. The use according to claim 4, wherein, the acidic amino acids comprise glutamic acid or aspartic acid.

7. The use according to claim 3, wherein, the protein in A2) comprises a protein having the amino acid sequence of SEQ ID No.1, SEQ ID No.2, SEQ ID No.3, or SEQ ID No.4.

8. The use according to claim 7, wherein, the substance is a biological material related to the protein, and the biological material comprises any one of the following:
B1) a nucleic acid molecule encoding the protein;
B2) an expression cassette containing the nucleic acid molecule of B1);
B3) a recombinant vector containing the nucleic acid molecule of B1), or a recombinant vector containing the expression cassette of B2);
B4) a recombinant microorganism containing the nucleic acid molecule of B1), or a recombinant microorganism containing the expression cassette of B2), or a recombinant microorganism containing the recombinant vector of B3);
or B5) a whole-cell catalyst containing the nucleic acid molecule of B1), or a whole-cell catalyst containing the expression cassette of B2), or a whole-cell catalyst containing the recombinant vector of B3), or a whole-cell catalyst containing the recombinant microorganism of B4).

9. The use according to claim 8, wherein, the nucleic acid molecule of B1) comprises any one of C1) to C6):
C1) a coding sequence comprising SEQ ID No.6;
C2) a coding gene comprising an amino acid sequence of SEQ ID No.1;
C3) a coding gene comprising an amino acid sequence of SEQ ID No.2;
C4) a coding gene comprising an amino acid sequence of SEQ ID No.3;
C5) a coding gene comprising an amino acid sequence of SEQ ID No.4;
or C6) a cdna molecule or dna molecule that hybridizes with the cdna or dna molecule defined by C1) or C2) or C3) or C4) or C5) and encodes a protein having the same function.

10. A protein, wherein, the protein comprises any one of the following:
A1) a protein having the amino acid sequence of SEQ ID No.5;
A2) a protein derived from a1) or having more than 45% identity with the protein shown in A1) and having the same function, obtained by substitution and/or deletion and/or addition of amino acid residues to the amino acid sequence shown in SEQ ID No.5;
or A3) a fusion protein having the same function obtained by linking a tag to the N-terminus and/or C-terminus of a1) or A2).

11. The protein according to claim 10, wherein, the protein in a2) comprises a protein having the amino acid sequence of SEQ ID No.1, SEQ ID No.2, SEQ ID No.3, or SEQ ID No.4.

12. A biological material, wherein, the biological material is a biological material related to the protein according to claim 10, and the biological material comprises any one of the following:
B1) a nucleic acid molecule encoding the protein;
B2) an expression cassette containing the nucleic acid molecule of B1);
B3) a recombinant vector containing the nucleic acid molecule of B1), or a recombinant vector containing the expression cassette of B2);
B4) a recombinant microorganism containing the nucleic acid molecule of B1), or a recombinant microorganism containing the expression cassette of B2), or a recombinant microorganism containing the recombinant vector of B3);
or B5) a whole-cell catalyst containing the nucleic acid molecule of B1), or a whole-cell catalyst containing the expression cassette of B2), or a whole-cell catalyst containing the recombinant vector of B3), or a whole-cell catalyst containing the recombinant microorganism of B4).

13. The biological material according to claim 12, wherein, the nucleic acid molecule of B1) comprises any one of the following:
C1) a coding sequence comprising SEQ ID No.6;
C2) a coding gene comprising an amino acid sequence of SEQ ID No.1;
C3) a coding gene comprising an amino acid sequence of SEQ ID No.3;
C4) a coding gene comprising an amino acid sequence of SEQ ID No.4;
C5) a coding gene comprising an amino acid sequence of SEQ ID No.5;
or C6) a cdna molecule or dna molecule that hybridizes with the cdna or dna molecule defined by C1) or C2) or C3) or C4) or C5) and encodes a protein having the same function.

14. A method for producing L-amino acids, wherein, it comprises fermenting and preparing L-amino acids using the biological material according to claim 12.

15. The method according to claim 14, wherein, the L-amino acid comprises L-glutamic acid, L-lysine, or L-threonine.

16. A method for producing L-amino acids, wherein, it comprises preparing L-amino acids using the protein according to claim 10.

17. The method according to claim 16, wherein, the L-amino acid comprises L-glutamic acid, L-lysine, or L-threonine.

18. Use of the protein according to claim 10 in the preparation of food, feed, medicine, fertilizer, or/and daily chemical products containing L-amino acids.

19. Use of the biological material according to claim 12 in the preparation of food, feed, medicine, fertilizer, or/and daily chemical products containing L-amino acids.
